# EUROPEAN PATENT SPECIFICATION

(11) **EP 4 536 686 B1**
(45) Date of publication and mention of the grant of the patent: **19.11.2025**
(21) Application number: 23728832.9
(22) Date of filing: 09.06.2023
(51) Int. Cl.: C07K 14/005, C12N 15/86

(54) **PEPTIDE-MODIFIED AAV CAPSID WITH ENHANCED MUSCLE TRANSDUCTION EFFICIENCY**
PEPTID-MODIFIZIERTES AAV-KAPSID MIT VERBESSERTER MUSKELTRANSDUKTIONSEFFIZIENZ
CAPSIDES AAV MODIFIÉS PAR DES PEPTIDES PRÉSENTANT UNE MEILLEURE EFFICACITÉ DE TRANSDUCTION MUSCULAIRE

(30) Priority: 10.06.2022 EP 22305845
(43) Date of publication of application: 16.04.2025
(73) Proprietor: GENETHON, 91000 Evry-Courcouronnes (FR); Institut National de la Santé et de la Recherche Médicale, 75013 Paris (FR); Universite d'Evry Val d'Essonne, 91000 Evry (FR)
(72) Inventor: RICHARD, Isabelle, 91100 Corbeil-Essonne (FR); HONG, Ai Vu, 912010 Draveil (FR)
(74) Representative: Plasseraud IP
(86) International application number: PCT/EP2023/065499
(87) International publication number: WO 2023/237748

(56) References cited:
- WO-A1-2019/193119
- WO-A1-2022/020616
- WO-A1-2022/053630
- DICARA D ET AL: "Structure-Function Analysis of Arg-Gly-Asp Helix Motifs in [alpha]v[beta]6 Integrin Ligands", JOURNAL OF BIOLOGICAL CHEMISTRY, vol. 282, no. 13, 30 March 2007 (2007-03-30), US, pages 9657 - 9665, XP055483044, ISSN: 0021-9258, DOI: 10.1074/jbc.M610461200
- DAVIES J A. ET AL: "Efficient Intravenous Tumor Targeting Using the [alpha]v[beta]6 Integrin-Selective Precision Virotherapy Ad5NULL-A20", vol. 13, no. 5, 1 January 2021 (2021-01-01), pages 864, XP055928338, Retrieved from the Internet <URL:https://www.ncbi.nlm.nih.gov/pmc/articles/PMC8151668/pdf/viruses-13-00864.pdf> DOI: 10.3390/v13050864

## Description

### FIELD OF THE INVENTION

The invention relates to a peptide-modified AAV capsid with enhanced muscle transduction efficiency. The invention relates also to the derived recombinant AAV vector particle packaging a gene of interest, and its use in gene therapy, in particular for treating muscle diseases.

### BACKGROUND OF THE INVENTION

Recombinant Adeno-Associated Virus (rAAV) vectors are widely used for *in vivo* gene transfer and clinical trials using AAV vectors are currently taking place for the treatment of a number of diseases.

AAV is a non-pathogenic virus belonging to the genus Dependoparvovirus within the family Parvoviridae. AAV is a non-enveloped virus composed of a capsid of about 26 nm in diameter and a single-stranded DNA genome of 4.7 kb. The genome carries two genes, rep and cap, flanked by two palindromic regions named Inverted terminal Repeats (ITR) that serve as the viral origins of replication and the packaging signal. The cap gene codes for three structural proteins VP1, VP2 and VP3 that compose the icosahedral AAV capsid through alternative splicing and translation from different start codons. VP1, VP2 and VP3 share the same C-terminal end which is all of VP3. Using AAV2 has a reference, VP1 has a 735 amino acid sequence (GenBank accession number YP_680426.1 accessed on 13 August 2018); VP2 (598 amino acids) starts at the Threonine 138 (T138) and VP3 (533 amino acids) starts at the methionine 203 (M203). The three different VPs contribute in a 1 VP1: 1 VP2: 10 VP3 ratio to the AAV2 capsid. The capsids of all AAV serotypes are assembled from 60 VP monomers with approximately 50 copies of VP3, 5 copies of VP 2 and 5 copies of VP1. The rep gene encodes four proteins required for viral replication Rep78, Rep68, Rep52 and Rep40. Recombinant AAV vectors encapsidate an ITR-flanked rAAV genome in which a therapeutic gene expression cassette replaces the AAV protein coding-sequences.

Tissue specificity is determined by the capsid serotype and commonly used AAV serotypes isolated from human and non-human primates can transduce specific organs more efficiently than others, such as AAV6, AAV8, AAV9 and AAV-rh74 in muscle tissue.

Libraries of AAV capsid variants displaying short random peptides on the surface of various AAV serotypes have been generated to screen for gene therapy vectors with altered cell specificities and/or transduction efficiencies (Review in Büning et al., Molecular Therapy: Methods & Clinical Development, 2019, 12, 248). AAV capsids comprising the insertion of a peptide comprising and RGD motif, which is known to bind several different cell-surface integrins, have been reported to improve gene delivery in muscle following systemic administration. The most efficient AAV capsids for muscle transduction in mice, non-human primates and/or human primary myotubes display the peptide RGDLGLS or P1 (AAVMYO or AAV9P1), RGDLTTP (MyoAAV 1A), GPGRGDQTTL (MyoAAV 2A), SNSRGDYNSL (MyoAAV 4A), ENRRGDFNNT (MyoAAV 4E), SAQRGDYVGL (MyoAAV 3A), QERRGDYTSM (MyoAAV 4C) inserted into the variable region VIII (WO 2019/207132; Weinmann et al., Nature communications, 2020, 11, 5432; Tabebordbar et al., Cell, 2021, 184, 4919-4938).

WO2022/020616 discloses the insertion of a peptide comprising a XmRGDXn motif into an AAV capsid in order to target a muscle cell. WO2022/053630 discloses a hybrid between AAV9 and AAVrh74 capsid proteins with increased muscle tropism due to insertion of the peptide RGDLGLS. WO2019/193119 discloses an AAV capsid protein comprising a muscle targeting insertion of peptide RGDLGLS. Dicara et al., Journal of Biological Chemistry, 2007, 282, 9657-9665, discloses RGDLXXL/I as binding motif for integrin heterodimer αVβ6 (IGAV-B6) which is significantly upregulated on many carcinomas. Davies et al., VIRUSES, 2021, 13, 864, discloses a tumor-selective adenovirus comprising an IGAV-B6 binding peptide comprising the motif RGDLXXL/I.

The ability to transduce muscle efficiently, selectively and safely with systemically-delivered AAV vectors would be beneficial for gene therapy of many human diseases.

### SUMMARY OF THE INVENTION

The inventors have shown that recombinant adeno-associated virus (AAV) capsid comprising the insertion of a peptide RGDLXXL/I with XX different from GS and ST (SEQ ID NO: 1), which binds to the integrin heterodimer alpha-αVβ6 (ITGAV-B6) has an increased transduction efficiency in muscle, particularly after systemic delivery of recombinant AAV vector.

Therefore, the invention relates to a recombinant adeno-associated virus (AAV) capsid protein, comprising the insertion of at least one copy of a peptide comprising the motif RGDLXXL/I with XX different from GS and ST (SEQ ID NO: 1), wherein said recombinant AAV capsid protein modified with the peptide has an increased transduction efficiency in muscle compared to the recombinant AAV capsid protein not modified with the peptide.

In some embodiments, the insertion is into the variable region IV.

In some embodiments, the recombinant AAV capsid protein is a hybrid between AAV serotype 9 and AAV serotype 74 capsid proteins.

In some preferred embodiments, the insertion is into position 452; preferably wherein the peptide insertion replaces the residues from positions 453 to 459, and the indicated positions being determined by alignment with SEQ ID NO: 20.

In some embodiments, said motif is RGDLX₁X₂L/I wherein X₁ is G, A, L, K or Q and X₂ is R, K, E, D, A, L, T, I or V. In some particular embodiments, said motif has a sequence selected from the group consisting of: RGDLGRL (SEQ ID NO: 2), RGDLGEL (SEQ ID NO: 3), RGDLATI (SEQ ID NO: 4), RGDLAEL (SEQ ID NO: 5), RGDLQVL (SEQ ID NO: 6) and RGDLAEI (SEQ ID NO: 7); preferably SEQ ID NO: 2 or SEQ ID NO: 4; more preferably SEQ ID NO: 2. In some particular embodiments, said peptide comprises a sequence selected from the group consisting of: TDGRGDLGRLGP (SEQ ID NO: 14), SEPRGDLGELNA (SEQ ID NO: 15), EPRRGDLATIGS (SEQ ID NO: 16), TDQRGDLAELHG (SEQ ID NO: 17), APVRGDLQVLAP (SEQ ID NO: 18) and APVRGDLAEINP (SEQ ID NO: 19); preferably SEQ ID NO: 14 or 16; more preferably SEQ ID NO: 14. In some more preferred embodiments, the recombinant AAV capsid protein comprises a sequence having at least 85% identity with any one of SEQ ID NO: 21 to 26; preferably SEQ ID NO: 21 or 23; more preferably SEQ ID NO: 21.

The invention also relates to a polynucleotide encoding the recombinant AAV capsid protein according to the present disclosure.

The invention further relates to a recombinant plasmid comprising the polynucleotide according to the present disclosure.

Another aspect of the invention relates to an AAV vector particle packaging a gene of interest, which comprises the recombinant AAV capsid protein according to the present disclosure. In some embodiments, the gene of interest is selected from the group consisting of: therapeutic genes; genes encoding therapeutic proteins or peptides such as therapeutic antibodies or antibody fragments and genome editing enzymes; and genes encoding therapeutic RNAs such as interfering RNAs, guide RNAs for genome editing and antisense RNAs capable of exon skipping.

Another aspect of the invention relates to a pharmaceutical composition comprising a therapeutically effective amount of AAV vector particle according to the present disclosure, or cell stably transduced by said AAV vector particle according to the present disclosure.

Another aspect of the invention relates to the pharmaceutical composition according to the present disclosure, for use as a medicament in gene therapy, preferably for use in the treatment of muscle diseases. In some preferred embodiments, the pharmaceutical composition targets a gene responsible for a muscle disease chosen from Dystrophinopathies and Limb-girdle muscular dystrophies; preferably a gene selected from the group comprising: *DMD, CAPN3, DYSF, FKRP, DNAJB6, ANO5, SGCA, SGCB* and *SGCG.*

### DETAILED DESCRIPTION OF THE INVENTION

### Modified AAV capsid protein

The invention relates to a recombinant adeno-associated virus (AAV) capsid protein comprising the insertion of at least one copy of a peptide comprising the motif RGDLXXL/I wherein XX is different from GS and ST (SEQ ID NO: 1), wherein said recombinant AAV capsid protein modified with the peptide has an increased transduction efficiency in muscle compared to the recombinant AAV capsid protein not modified with the peptide.

Integrins are a class of adhesion receptors comprising 18 alpha subunits and 8 beta subunits that form 24 different integrin heterodimers (Hynes, Cell, 2002, 110, 673-687). The motif RGDLXXL/I wherein XX may be any pair of amino acids except GS and ST (SEQ ID NO: 1), binds with high affinity to the integrin heterodimer αVβ6 (ITGAV-B6) which is highly enriched in the skeletal muscle tissue and upregulated in dystrophic muscle as shown in the examples of the present application. In this motif, LxxL/I forms an amphipathic α-helix that binds in a hydrophobic pocket in the B6 subunit. The motif can be found in the prodomains of TGF-β1, TGF-β3, and FMDV virus capsid, which all specifically bind to ITGAV-B6 (Dong et al., Nature Struct. Mol. Biol., 2014, 21, 1091-1096; Dong et al., Nature, 2017, 542, 55-59; Kotecha et al., Nature communications, 2017, 8, 15408; Protein Data Bank accession number 5FFO,4UM9 and 5NEM). Therefore, the peptide comprising the motif RGDLXXL/I of SEQ ID NO: 1 according to the invention binds to muscle cells and directs or targets capsid-modified rAAV vectors carrying the peptide to muscle cells and tissue *in vivo,* in particular in subjects suffering from muscle disease.

The modified AAV capsid protein according to the invention is a functional AAV capsid which is able to form recombinant AAV vector particles which transduce muscle cell, tissue or organ *in vitro* or *in vivo.* Furthermore, the modified AAV capsid protein according to the invention has an increased transduction efficiency in muscle compared to the corresponding unmodified AAV capsid protein from which it is derived.

The superior transduction efficiency of muscle by the modified AAV capsid protein according to the invention may be determined by measuring the ability of AAV vector particles comprising the modified AAV capsid protein to transduce muscle cell, tissue or organ *in vitro* or *in vivo* using standard assays that are well-known in the art such as those disclosed in the examples of the present application. AAV vector transduction may be determined *in vitro* or *in vivo* by measuring vector genome copy number or transgene expression. Vector genome copy number per diploid genome may be measured by standard assays that are well known in the art such as real-time PCR assay. Transgene expression is advantageously measured using a reporter gene such as luciferase or fluorescent protein (GFP or others) by standard assays that are well known in the art such as *in vivo* or *in vitro* quantitative bioluminescence or fluorescence assays *in vivo* or *in vitro.* For example, muscle transduction level may be determined by local or systemic administration of AAV vector particles carrying the modified AAV capsid protein in animal models such as mouse models that are well known in the art and disclosed in the examples of the present application. AAV vectors comprising the corresponding unmodified AAV capsid protein are used for comparison.

An increased transduction efficiency in muscle level refers to an increased level (higher level or elevated level) of transduction in muscle, in particular to a transgene expression level that is increased in at least one muscle cell, tissue or organ, by at least 1.5 fold, preferably 3, 5, 10, 50, 100 folds or more compared to unmodified AAV capsid protein or a vector copy number that is increased in at least one muscle cell, tissue or organ, by at least 1.1, fold, preferably 1.5, 2, 2.5, 3, 3.5 folds or more compared to unmodified AAV capsid protein. As a result of higher transduction level in muscle, the modified AAV capsid protein according to the invention has an improved biodistribution. This means that it targets significantly better muscle tissue or organ without increasing the targeting of other (non-target) tissues, in particular the liver (i.e. improved specificity).

As used herein, the term "muscle" refers to cardiac muscle (i.e. heart) and skeletal muscle. The term "muscle cells" refers to myocytes, myotubes, myoblasts, and/or satellite cells. The skeletal muscles are classified in different groups based on their anatomical position in the body. The transduction efficiency or tropism of the modified AAV capsid according to the invention in different skeletal muscle groups may be measured in mice *Tibialis* (TA), *Extensor Digitorum Longus* (EDL), *Quadriceps* (Qua), *Gastrocnemius* (Ga), *Soleus* (Sol), Triceps, Biceps and/or Diaphragm; in particular in mice *Tibialis* (TA), Diaphragm and/or *Quadriceps* (Qua) muscles. The expression "muscle transduction" refers to the tropism for the heart and the various skeletal muscles present in the body.

In the following description, the amino acid residues are designated by the standard one letter amino acid code.

"a", "an", and "the" include plural referents, unless the context clearly indicates otherwise. As such, the term "a" (or "an"), "one or more" or "at least one" can be used interchangeably herein; unless specified otherwise, "or" means "and/or".

A modified AAV capsid protein according to the invention is a recombinant protein.

As used herein, "a sequence" refers to 1 amino acid or at least 2 consecutive amino acids.

In the present description, an insertion at or into a given position of AAV capsid protein sequence refers to an insertion after the amino acid residue at that position.

The term "identity" refers to the sequence similarity between two polypeptide molecules or between two nucleic acid molecules. When a position in both compared sequences is occupied by the same base or same amino acid residue, then the respective molecules are identical at that position. The percentage of identity between two sequences corresponds to the number of matching positions shared by the two sequences divided by the number of positions compared and multiplied by 100. Generally, a comparison is made when two sequences are aligned to give maximum identity. The identity may be calculated by alignment using, for example, the GCG (Genetics Computer Group, Program Manual for the GCG Package, Version 7, Madison, Wisconsin) pileup program, or any of sequence comparison algorithms such as BLAST, FASTA or CLUSTALW.

In some embodiments, the modified AAV capsid according to the invention increases the transduction level in muscle after systemic delivery of the AAV vector.

In some embodiments, the RGDLXXL/I motif according to the invention consists of RGDLX₁X₂L/I wherein X₁ is G, A, L, K or Q and X₂ is R, K, E, D, A, L, T, I or V. This motif was identified by modelling the sequences that can enhance binding of RGDLXXL/I motif toward ITGAV-B6 or maintain stability of the capsid protein. In some particular embodiments, the RGDLXXL/I motif according to the invention consists of a sequence selected from the group consisting of : RGDLGRL (SEQ ID NO: 2), RGDLGEL (SEQ ID NO: 3), RGDLATI (SEQ ID NO: 4), RGDLAEL (SEQ ID NO: 5), RGDLQVL (SEQ ID NO: 6) and RGDLAEI (SEQ ID NO: 7); preferably SEQ ID NO: 2 or SEQ ID NO: 4; more preferably SEQ ID NO: 2. In some particular embodiments, the peptide comprises the Z-RGDLXXL/I motif according to the invention wherein Z is G, P, Q , V or R; preferably G or R. In particular embodiments, the peptide comprises a sequence selected from the group consisting of: GRGDLGRL (SEQ ID NO: 8), PRGDLGEL (SEQ ID NO: 9), RRGDLATI (SEQ ID NO: 10), QRGDLAEL (SEQ ID NO: 11), VRGDLQVL (SEQ ID NO: 12) and VRGDLAEI (SEQ ID NO: 13); preferably SEQ ID NO: 8 or 10; more preferably SEQ ID NO: 8.

In some embodiments, the RGDLXXL/I or Z-RGDLXXL/I motif according to the invention is flanked in N-ter and/or C-ter end(s) by a sequence of up to five amino acids (1, 2, 3, 4 or 5) which may be the same or different; preferably by a sequence of 2 amino acids which may be the same or different. According to the present invention, the sequence in N-ter and C-ter end of the motif is a flanking sequence directly adjacent to the N-ter and C-ter end of the motif. The N-terminal and C-terminal flanking sequences are advantageously selected from the group consisting of: TD, GP, EP and GS; preferably TD or EP in N-ter and GP or GS in C-ter. In some particular embodiments, the peptide comprises or consists of a sequence selected from the group consisting of: TDGRGDLGRLGP (SEQ ID NO: 14), SEPRGDLGELNA (SEQ ID NO: 15), EPRRGDLATIGS (SEQ ID NO: 16) TDQRGDLAELHG (SEQ ID NO: 17), APVRGDLQVLAP (SEQ ID NO: 18) and APVRGDLAEINP (SEQ ID NO: 19);preferably SEQ ID NO: 14 or 16; more preferably SEQ ID NO: 14. The peptide comprising the RGDLXXL/I motif according to the invention consists generally of a sequence of up to 30 amino acids. The peptide may consist of 9, 10, 11, 12, 13, 14, 15, 16, 17, 18, 19, 20, 21, 22, 23, 24, 25, 26, 27, 28, 29, 30 amino acids. In some embodiments, the peptide consists of a sequence of up to 25, 20 or 15 amino acids. Preferably, the targeting peptide consists of a sequence of 12, 13, 14 or 15 amino acids; more preferably 12 amino acids.

In some embodiments, the modified AAV capsid protein according to the invention comprises up to 5 copies (1, 2, 3, 4 or 5) of the peptide comprising the RGDLXXL/I motif according to the invention.

The one or more peptides comprising the RGDLXXL/I motif according to the invention are inserted into site(s) exposed on the AAV capsid surface. Sites on the AAV capsid surface which are exposed on the capsid surface and tolerate peptide insertions, i.e. do not affect assembly and packaging of the virus capsid, are well-known in the art and include in particular the variable regions (VRs) which form loops at the top of the protrusions, such as VR-IV, -V and -VIII (Review in Büning et al., Molecular Therapy: Methods & Clinical Development, 2019, 12, 248-). VR-IV corresponds to Y445 to A476 (broad definition) or Q451 to L462 (narrow definition); VR-V corresponds to C485 to G515 (broad definition) or R490 to T509 (narrow definition); VR-VIII corresponds to I581 to L604 (broad definition) or L586 to I595 (narrow definition) according to the numbering in AAV8 capsid protein sequence. The peptide insertion site is advantageously at a site of the common VP3 region exposed on the AAV capsid surface such as for example position 587, 588, 589, 453, 520 (combined with 584), 584 and 585, according to the numbering in AAV2 capsid protein sequence. The peptide insertion sites are indicated by reference to AAV2 or AAV8 capsid amino acid sequence. AAV2 capsid (VP1) protein sequence corresponds to GenBank accession number YP_680426.1 accessed on 13 August 2018. AAV8 capsid (VP1) protein sequence corresponds to GenBank accession number YP_077180.1 accessed on 13 August 2018. After sequence alignment of any other AAV capsid sequence with AAV2 or AAV8 capsid sequence using standard protein sequence alignment programs that are well-known in the art, such as for example BLAST, FASTA, CLUSTALW, and the like, a person skilled in the art can easily obtained the corresponding positions of the peptide insertion sites in other AAV capsid sequences. Insertion sites in VR-VIII include position 590 in AAV1; positions 587 or 588 in AAV2; position 586 in AAV3 or AAV4; position 575 in AAV5; position 585 in AAV6; positions 585 or 590 in AAV8; positions 588 or 589 in AAV9; position 589 in AAV9.rh74.

In some preferred embodiments, the modified AAV capsid according to the invention comprises at least a RGDLXXL/I peptide insertion into the variable region IV (VR-IV) or the variable region VIII (VR-VIII) preferably into the VR-IV. Preferably, the insertion in VR-IV is into position 450 (T450) according to the numbering in AAV2 capsid sequence; the corresponding position is 452 (S452) in AAV9.rh74 hybrid capsid protein sequence (SEQ ID NO: 8). The insertion in VR-VIII is preferably at position 587 (N587) according to the numbering in AAV2 capsid protein; this position is 589 (N589) in AAV9.rh74 capsid protein sequence.

The RGDLXXL/I peptide may be inserted between 2 consecutive amino acids of the AAV capsid protein sequence (no deletion) or may replace some or all of the residue(s) from the insertion site (deletion). In some embodiments, the RGDLXXL/I peptide replaces the residues from the insertion site, in particular the residues from positions 451 to 457 of AAV2 capsid protein sequence, corresponding to the residues from positions 453 to 459 of AAV9.rh74 capsid protein sequence.

The modified AAV capsid protein may be derived from any natural or artificial AAV capsid serotype including hybrid serotypes and variant serotypes. Non-limiting examples of AAV capsid serotypes from which the modified AAV capsid protein may be derived include AAV1, AAV2, AAV3 (including types 3A and 3B), AAV4, AAV5, AAV6, AAV7, AAV8, AAV9, AAV10, AAV11, AAV12, AAV2i8, AAVrh10, AAVrh39, AAVrh43, AAVrh74, AAV-LK03, AAV2G9, AAV.PHP, AAV-Anc80, AAV3B and hybrid between AAV9 and AAVrh74. In some embodiments, the modified AAV capsid protein is from AAV serotype selected from the group consisting of: AAV6, AAV8, AAV9, AAVrh74 and hybrid between AAV9 and AAVrh74. In some preferred embodiments, the modified AAV capsid protein is from AAV8, AAV9 or hybrid between AAV9 and AAVrh74; or from AAV9 or hybrid between AAV9 and AAVrh74, still more preferably hybrid between AAV9 and AAVrh74. AAV9 capsid corresponds in particular to the amino acid sequence GenBank accession number AY530579.1 accessed on 24 June 2004. The hybrid between AAV9 and AAVrh74 is preferably AAV9.rh74 as disclosed in WO2019/193119; more preferably AAV9.rh74 capsid having the amino acid sequence SEQ ID NO: 20.

In some preferred embodiments, the modified AAV capsid protein is from AAV9.rh74 serotype and comprises the peptide containing the RGDLXXLII motif according to the invention inserted into position 452 and replacing the residues from positions 453 to 459 of AAV9.rh74 capsid protein sequence. In some preferred embodiments, the peptide comprises or consists of a sequence selected from the group consisting of: SEQ ID NO: 2 to 19, preferably SEQ ID NO: 2, 4, 8, 10, 14 or 16; still more preferably SEQ ID NO: 2, 8 or 14.

In some more particular embodiments, the modified AAV capsid protein, comprises or consists of a sequence having at least 85%, 87%, 88%, 90%, 95%, 97%, 98% or 99% identity with any one of SEQ ID NO: 21 to 26; preferably SEQ ID NO: 21 or 23; more preferably which comprises or consists of the sequence of SEQ ID NO: 21 or 23; still more preferably SEQ ID NO: 21. The variant according to the invention has no mutations in the peptide containing the RGDLXXL/I motif or Z-RGDLXXL/I motif according to the invention and preferably also in the 5 amino acid sequence; preferably the 10 amino acid sequence before and after the insertion site.

In some embodiments, the modified AAV capsid protein is a modified VP1, VP2 or VP3 protein. In some particular embodiments, the modified VP1, VP2 or VP3 protein is derived from any one SEQ ID NO: 21 to 26; preferably SEQ ID NO: 21 or 23; more preferably SEQ ID NO: 21. VP2 corresponds to the amino acid sequence from T138 to the end of any one SEQ ID NO: 21 to 26. VP3 corresponds to the amino acid sequence from M204 to the end of any one of SEQ ID NO: 21 to 26.

In some embodiments, the modified AAV capsid protein is a chimeric VP1 or VP2 protein comprising the peptide insertion into the common VP3 region of an AAV serotype and VP1-specific and/or VP2-specific N-terminal region(s) from other AAV serotype(s) (e.g. AAV serotype(s) different from the serotype of the VP3 region). In some particular embodiments, the chimeric VP1 or VP2 protein is derived from SEQ ID NO: 9 or 10.

In the various embodiments of the invention, the amino acid sequence of the modified AAV capsid protein may be advantageously selected to have lowest estimated energy using appropriate methods that are available in the art such as Rosetta energy function for macromolecular modeling and design (review in Alford et al., J. Chem. Theory Comput, 2017, 13, 6, 3031-3048). For example, Rosetta energy function is used to sample exhaustively low-energy structure-sequence pair. This method ensures that the designed modified AAV capsid remains stable and therefore can maintain or even improve AAV vector productivity.

### Polynucleotide, vector, and use for AAV vector production

Another aspect of the invention is a polynucleotide encoding the recombinant modified AAV capsid protein in expressible form. The polynucleotide may be DNA, RNA or a synthetic or semi-synthetic nucleic acid.

In some embodiments, the polynucleotide encodes a modified AAV9.rh74 capsid protein according to the invention, in particular a modified AAV9.rh74 capsid protein comprising a peptide comprising or consisting of a sequence selected from the group consisting of: SEQ ID NO: 2 to 19, preferably SEQ ID NO: 2, 4, 8, 10, 14 or 16; still more preferably SEQ ID NO: 2, 8 or 14

In some preferred embodiments, the polynucleotide encodes a modified AAV9.rh74 capsid protein comprising or consisting of a sequence having at least 85%, 87%, 88%, 90%, 95%, 97%, 98% or 99% identity with any one SEQ ID NO: 21 to 26; preferably SEQ ID NO: 21 or 23; still more preferably SEQ ID NO: 21.

In some more preferred embodiments, the polynucleotide comprises a sequence having at least 80%, 85%, 90%, 95%, 97%, 98% or 99% identity with any one of SEQ ID NO: 27 to 32; preferably SEQ ID NO: 27 or 29; more preferably SEQ ID NO: 27. The nucleotide sequence SEQ ID NO: 27 encodes the modified AAV9.rh74 capsid protein of SEQ ID NO: 21. The nucleotide sequence SEQ ID NO: 29 encodes the modified AAV9.rh74 capsid protein of SEQ ID NO: 23. The polynucleotide is a functional polynucleotide sequence, which means that the sequence of the polynucleotide codes for the modified AAV capsid protein according to the invention.

In some embodiments, the polynucleotide further encodes AAV Replicase (Rep) protein in expressible form, preferably Rep from AAV2.

The polynucleotide is advantageously inserted into a recombinant vector, which includes, in a non-limiting manner, linear or circular DNA or RNA molecules consisting of chromosomal, non-chromosomal, synthetic or semi-synthetic nucleic acids, such as in particular viral vectors, plasmid or RNA vectors. Numerous vectors into which a nucleic acid molecule of interest can be inserted in order to introduce it into and maintain it in a eukaryotic host cell are known *per se;* the choice of an appropriate vector depends on the use envisioned for this vector (for example, replication of the sequence of interest, expression of this sequence, maintaining of this sequence in extrachromosomal form, or else integration into the chromosomal material of the host), and also on the nature of the host cell.

In some embodiments, the vector is a plasmid.

The recombinant vector for use in the present invention is an expression vector comprising appropriate means for expression of the modified AAV capsid protein (AAV Cap), and maybe also AAV Rep protein. Usually, each coding sequence (modified AAV Cap and AAV Rep) is inserted into a separate expression cassette either in the same vector or separately. Each expression cassette comprises the coding sequence (open reading frame or ORF) functionally linked to the regulatory sequences which allow the expression of the corresponding protein in AAV producer cells, such as in particular promoter, promoter/enhancer, initiation codon (ATG), stop codon, transcription termination signal. Alternatively, the modified AAV Cap and the AAV Rep proteins may be expressed from a unique expression cassette using an Internal Ribosome Entry Site (IRES) inserted between the two coding sequences or a viral 2A peptide. In addition, the codon sequences encoding the modified AAV Cap, and AAV Rep if present, are advantageously optimized for expression in AAV producer cells, in particular human producer cells.

Another aspect is a cell stably transformed with a recombinant vector for expression of the modified AAV capsid protein, and preferably also of the AAV Rep protein. The cell stably expresses the modified AAV capsid and AAV Rep proteins (producer cell line). The producer cell is advantageously a human cell.

The vector, preferably a recombinant plasmid, or the cell are useful for producing hybrid AAV vectors comprising the hybrid AAV capsid protein of the invention, using standard AAV production methods that are well-known in the art (Review in Aponte-Ubillus et al., Applied Microbiology and Biotechnology, 2018, 102: 1045-1054).

AAV vectors are usually produced by co-transfecting cells suitable for AAV production with a plasmid containing recombinant AAV vector genome comprising the gene of interest inserted into an expression cassette, flanked by AAV ITRs (AAV transfer plasmid), and plasmid(s) expressing AAV Rep and Cap proteins. Alternatively, producer cells according to the invention (which stably express AAV Rep and Cap proteins) may be transfected with an AAV transfer plasmid.

Briefly, following transfection with above plasmid(s) in the presence of sufficient helper function to permit packaging of the rAAV vector genome into AAV capsid particle, the cells are incubated for a time sufficient to allow the production of AAV vector particles, the cells are then harvested, lysed, and AAV vector particles are purified by standard purification methods such as affinity chromatography and Iodixanol or Cesium Chloride density gradient ultracentrifugation.

### AAV particle, cell

Another aspect of the invention is an AAV particle comprising the modified recombinant AAV capsid protein of the invention.

The AAV particle comprises modified VP1, VP2 and/or VP3 capsid proteins according to the present invention. In some embodiments, the AAV particle comprises modified VP1, VP2 and VP3 capsid proteins of the same serotype. In some embodiments, the AAV particle further or alternatively comprises chimeric VP1 and/or VP2 capsid proteins and a modified VP3 protein . In some embodiments, the AAV particle is a mosaic AAV particle further comprising another AAV capsid protein from a natural or artificial AAV serotype other than the serotype(s) of the modified AAV capsid including chimeric modified AAV capsid, wherein the mosaic AAV particle has an increased muscle transduction efficiency or level. An artificial AAV serotype may be with no limitation, a chimeric AAV capsid, a recombinant AAV capsid, or a humanized AAV capsid. Such an artificial capsid may be generated by any suitable technique, using a selected AAV sequence (e.g. a fragment of a VP1 capsid protein) in combination with heterologous sequences which may be obtained from a different selected AAV serotype, non-contiguous portions of the same AAV serotype, from a non-viral AAV source or from a non-viral source.

Preferably, the AAV particle is a recombinant AAV (rAAV) vector particle. The AAV vector particle is suitable for gene therapy directed to muscle cells, tissue or organ in the individual. The rAAV vector particle is packaging a gene of interest. The genome of the rAAV vector may either be a single-stranded or self-complementary double-stranded genome (McCarty et al, Gene Therapy, 2003, Dec., 10(26), 2112-2118). Self-complementary vectors are generated by deleting the terminal resolution site (trs) from one of the AAV terminal repeats. These modified vectors, whose replicating genome is half the length of the wild-type AAV genome have the tendency to package DNA dimers. The AAV genome is flanked by ITRs. In particular embodiments, the AAV vector is a pseudotyped vector, i.e. its genome and capsid are derived from AAVs of different serotypes. In some preferred embodiments, the genome of the pseudotyped vector is derived from AAV2. The rAAV vector particle may be obtained using standard AAV production methods that are well-known in the art as disclosed above.

By "gene of interest", it is meant a gene useful for a particular application, such as with no limitation, diagnosis, reporting, modifying, therapy and genome editing.

For example, the gene of interest may be a therapeutic gene, a reporter gene or a genome-editing enzyme.

By "gene of interest for therapy", "gene of therapeutic interest", or "heterologous gene of interest", it is meant a therapeutic gene or a gene encoding a therapeutic protein, peptide or RNA.

The gene of interest is any nucleic acid sequence capable of modifying a target gene or target cellular pathway, in cells of target organ (i.e. muscle). For example, the gene may modify the expression, sequence or regulation of the target gene or cellular pathway. In some embodiments, the gene of interest is a functional version of a gene or a fragment thereof. The functional version of said gene includes the wild-type gene, a variant gene such as variants belonging to the same family and others, or a truncated version, which preserves the functionality of the encoded protein at least partially. A functional version of a gene is useful for replacement or additive gene therapy to replace a gene, which is deficient or non-functional in a patient. In other embodiments, the gene of interest is a gene which inactivates a dominant allele causing an autosomal dominant genetic disease. A fragment of a gene is useful as recombination template for use in combination with a genome editing enzyme.

Alternatively, the gene of interest may encode a protein of interest for a particular application, (for example an antibody or antibody fragment, a genome-editing enzyme) or a RNA. In some embodiments, the protein is a therapeutic protein including a therapeutic antibody or antibody fragment, or a genome-editing enzyme. In some embodiments, the RNA is a therapeutic RNA.

In some embodiments, the sequence of the gene of interest is optimized for expression in the treated individual, preferably a human individual. Sequence optimization may include a number of changes in a nucleic acid sequence, including codon optimization, increase of GC content, decrease of the number of CpG islands, decrease of the number of alternative open reading frames (ARFs) and/or decrease of the number of splice donor and splice acceptor sites.

The gene of interest is a functional gene able to produce the encoded protein, peptide or RNA in the target cells of the disease (i.e. muscle cells). In some embodiments, the gene of interest is a human gene. The AAV viral vector comprises the gene of interest in a form expressible in cells of target organs (i.e. muscle cells), including cardiac and skeletal muscle cells muscles. In particular, the gene of interest is operably linked to appropriate regulatory sequences for expression of a transgene in the individual's target cells, tissue(s) or organ(s). Such sequences which are well-known in the art include in particular a promoter, and further regulatory sequences capable of further controlling the expression of a transgene, such as without limitation, enhancer, terminator, intron, silencer, in particular tissue-specific silencer, and microRNA. The gene of interest is operably linked to a ubiquitous, tissue-specific or inducible promoter which is functional in cells of target organs (i.e. muscle(s)). The gene of interest may be inserted into an expression cassette further comprising additional regulatory sequences as disclosed above.

Examples of ubiquitous promoters include the CAG promoter, phosphoglycerate kinase 1 (PGK) promoter, the cytomegalovirus enhancer/promoter (CMV), the SV40 early promoter, the retroviral Rous sarcoma virus (RSV) LTR promoter, the dihydrofolate reductase promoter, the β-actin promoter, and the EF 1 promoter. Muscle-specific promoters include without limitation, upstream sequences derived from the desmin (Des) promoter, muscle creatine kinase (MCK) promoter, , alpha-myosin heavy chain (alpha-MHC) promoter, myosin light chain 2 (MLC-2) promoter, cardiac troponin C (cTnC) promoter, the human skeletal actin (HSA) promoter or synthetic muscle-specific promoters such as SpC5-12 promoter, CK6 promoter.

The RNA is advantageously complementary to a target DNA or RNA sequence or binds to a target protein. For example, the RNA is an interfering RNA such as a shRNA, a microRNA, a guide RNA (gRNA) for use in combination with a Cas enzyme or similar enzyme for genome editing, an antisense RNA capable of exon skipping such as a modified small nuclear RNA (snRNA) or a long non-coding RNA. The interfering RNA or microRNA may be used to regulate the expression of a target gene involved in muscle disease. The guide RNA in complex with a Cas enzyme or similar enzyme for genome editing may be used to modify the sequence of a target gene, in particular to correct the sequence of a mutated/deficient gene or to modify the expression of a target gene involved in a disease, in particular a neuromuscular disease. The antisense RNA capable of exon skipping is used in particular to correct a reading frame and restore expression of a deficient gene having a disrupted reading frame. In some embodiments, the RNA is a therapeutic RNA.

The genome-editing enzyme is any enzyme or enzyme complex capable of modifying a target gene or target cellular pathway, in particular in muscle cells. For example, the genome-editing enzyme may modify the expression, sequence or regulation of the target gene or cellular pathway. The genome-editing enzyme is advantageously an engineered nuclease, such as with no limitations, a meganuclease, zinc finger nuclease (ZFN), transcription activator-like effector-based nuclease (TALENs), Cas enzyme from clustered regularly interspaced palindromic repeats (CRISPR)-Cas system and similar enzymes. The genome-editing enzyme, in particular an engineered nuclease such as Cas enzyme and similar enzymes, may be a functional nuclease which generates a double-strand break (DSB) or single-stranded DNA break (nickase such as Cas9(D10A) in the target genomic locus and is used for site-specific genome editing applications, including with no limitations: gene correction, gene replacement, gene knock-in, gene knock-out, mutagenesis, chromosome translocation, chromosome deletion, and the like. For site-specific genome editing applications, the genome-editing enzyme, in particular an engineered nuclease such as Cas enzyme and similar enzymes may be used in combination with a homologous recombination (HR) matrix or template (also named DNA donor template) which modifies the target genomic locus by double-strand break (DSB)-induced homologous recombination. In particular, the HR template may introduce a transgene of interest into the target genomic locus or repair a mutation in the target genomic locus, preferably in an abnormal or deficient gene causing a a muscle disorder, such as for example a neuromuscular disease. Alternatively, the genome-editing enzyme, such as Cas enzyme and similar enzymes may be engineered to become nuclease-deficient and used as DNA-binding protein for various genome engineering applications such as with no limitation: transcriptional activation, transcriptional repression, epigenome modification, genome imaging, DNA or RNA pull-down and the like.

The disclosure also relates to an isolated cell, in particular a cell from an individual, which is stably transduced with a rAAV vector particle of the invention. The individual is advantageously a patient to be treated. In some embodiments, the cell is a muscle cell according to the present disclosure, a progenitor of said cell or a pluripotent stem cell such as induced pluripotent stem cell (iPS cell), embryonic stem cells, fetal stem cell and adult stem cell.

### Pharmaceutical composition and therapeutic uses

Another aspect of the invention is a pharmaceutical composition comprising at least an active agent selected from an AAV vector particle or a cell of the present disclosure, and a pharmaceutically acceptable carrier.

The rAAV vector particle, cell and derived pharmaceutical composition may be used for treating diseases by gene therapy, in particular targeted gene therapy directed to muscle cells or tissue. The cell and derived pharmaceutical composition may be used for treating diseases by cell therapy, in particular cell therapy directed to muscle cells.

As used herein "Gene therapy" refers to a treatment of an individual which involves delivery of nucleic acid of interest into an individual's cells for the purpose of treating a disease. Delivery of the nucleic acid is generally achieved using a delivery vehicle, also known as a vector. The rAAV vector particle of the invention may be employed to deliver a gene to a patient's cells.

As used herein "Cell therapy" refers to a process wherein cells stably transduced by a rAAV vector particle of the invention are delivered to the individual in need thereof by any appropriate mean such as for example by intravenous injection (infusion), or injection in the tissue of interest (implantation or transplantation). In particular embodiments, cell therapy comprises collecting cells from the individual, transducing the individual's cells with the rAAV vector particle of the invention, and administering the stably transduced cells back to the patient. As used herein "cell" refers to isolated cell, natural or artificial cellular aggregate, bioartificial cellular scaffold and bioartificial organ or tissue.

Gene therapy can be performed by gene transfer, gene editing, exon skipping, RNA-interference, trans-splicing or any other genetic modification of any coding or regulatory sequences in the cell, including those included in the nucleus, mitochondria or as commensal nucleic acid such as with no limitation viral sequences contained in cells.

The two main types of gene therapy are the following:
- a therapy aiming to provide a functional replacement gene for a deficient/abnormal gene: this is replacement or additive gene therapy;
- a therapy aiming at gene or genome editing: in such a case, the purpose is to provide to a cell the necessary tools to correct the sequence or modify the expression or regulation of a deficient/abnormal gene so that a functional gene is expressed or an abnormal gene is suppressed (inactivated): this is gene editing therapy.

**In** additive gene therapy, the gene of interest may be a functional version of a gene, which is deficient or mutated in a patient, as is the case for example in a genetic disease. In such a case, the gene of interest will restore the expression of a functional gene. Thus, by gene editing or gene replacement a correct version of this gene is provided in target cells (i.e. muscle cells) of affected patients, this may contribute to effective therapies against the disease.

Gene or genome editing uses one or more gene(s) of interest, such as:
- a gene encoding a therapeutic RNA as defined above such as an interfering RNA like a shRNA or a microRNA, a guide RNA (gRNA) for use in combination with a Cas enzyme or similar enzyme, or an antisense RNA capable of exon skipping such as a modified small nuclear RNA (snRNA); and
- a gene encoding a genome-editing enzyme as defined above such as an engineered nuclease like a meganuclease, zinc finger nuclease (ZFN), transcription activator-like effector-based nuclease (TALENs), Cas enzyme or similar enzymes; or a combination of such genes, and maybe also a fragment of a functional version of a gene for use as recombination template, as defined above.

Gene therapy is used for treating muscle diseases. Muscle diseases include various inherited (genetic) and acquired diseases or disorders affecting the structure or function of muscle including skeletal and cardiac muscle. The diseases may be caused by trauma, infection, degeneration, structural or metabolic defects, tumors, inflammatory or autoimmune disorders, or other causes. Non-limiting examples of muscle diseases that can be treated by gene therapy include neuromuscular genetic disorders such as muscular genetic disorders; cancer and auto-immune diseases.

**In** some embodiments, the target gene for gene therapy (additive gene therapy or gene editing) is a gene responsible for a neuromuscular disease. Neuromuscular genetic disorders include in particular: Muscular dystrophies, Congenital muscular dystrophies, Congenital myopathies, Distal myopathies, Other myopathies, Myotonic syndromes, Ion Channel muscle diseases, Malignant hyperthermia, Metabolic myopathies, Hereditary Cardiomyopathies, Congenital myasthenic syndromes, Motor Neuron diseases, Hereditary paraplegia, Hereditary motor and sensory neuropathies and other neuromuscular disorders.

Particular examples of neuromuscular genetic disorders that can be treated using a capsid-modified rAAV according to the invention are listed below:
- Dystrophinopathies are a spectrum of X-linked muscle diseases caused by pathogenic variants in *DMD* gene, which encodes the protein dystrophin. Dystrophinopathies comprises Duchenne muscular dystrophy (DMD), Becker muscular dystrophy (BMD) and DMD-associated dilated cardiomyopathy.
- The Limb-girdle muscular dystrophies (LGMDs) are a group of disorders that are clinically similar to DMD but occur in both sexes as a result of autosomal recessive and autosomal dominant inheritance. Limb-girdle dystrophies are caused by mutation of genes that encode sarcoglycans and other proteins associated with the muscle cell membrane, which interact with dystrophin. The term LGMD1 refers to genetic types showing dominant inheritance (autosomal dominant), whereas LGMD2 refers to types with autosomal recessive inheritance. Pathogenic variants at more than 50 loci have been reported (LGMD1A to LGMD1G; LGMD2A to LGMD2W). Calpainopathy (LGMD2A) is caused by mutation of the gene *CAPN3* with more than 450 pathogenic variants described. Contributing genes to LGMD phenotype include: anoctamin 5 (*ANO5*)*,* blood vessel epicardial substance *(BVES),* calpain 3 *(CAPN3),* caveolin 3 *(CAV3),* CDP-L-ribitol pyrophosphorylase A *(CRPPA),* dystroglycan 1 (*DAG1*)*,* desmin *(DES),* DnaJ heat shock protein family (Hsp40) homolog, subfamily B, member 6 *(DNAJB6),* dysferlin *(DYSF),* fukutin related protein (*FKRP*), fukutin *(FKT),* GDP-mannose pyrophosphorylase B (*GMPPB*), heterogeneous nuclear ribonucleoprotein D like (*HNRNPDL*)*,* LIM zinc finger domain containing 2 (*LIMS2*)*,* lain A:C (*LMNA*)*,* myotilin *(MYOT),* plectin *(PLEC),* protein O-glucosyltransferase 1 *(PLOGLUT1),* protein O-linked mannose N-acetylglucosaminyltransferase 1 (beta 1,2-) (*POMGNT1*)*,* protein O-mannose kinase *(POMK),* protein O-mannosyltransferase 1 *(POMT1),* protein O-mannosyltransferase 2 *(POMT2),* sarcoglycan alpha *(SGCA),* sarcoglycan beta *(SGCB),* sarcoglycan delta *(SGCD),* sarcoglycan gamma *(SGCG),* titin-cap *(TCAP),* transportin 3 (*TNPO3*)*,* torsin 1A interacting protein (*TOR1AIP1*)*,* trafficking protein particle complex 11 (*TRAPPC11*)*,* tripartite motif containing 32 (TRIM 32) and titin (TTN). Major contributing genes to LGMD phenotype include *CAPN3, DYSF, FKRP* and *ANO5* (Babi Ramesh Reddy Nallamilli et al., Annals of Clinical and Translational Neurology, 2018, 5, 1574-1587.
- The Emery-Dreifuss Muscular Dystrophy (EDMD) caused by defects in one of the gene including the *EMD* gene (coding for emerin), the *FHL1* gene and the *LMNA* gene (encoding lamin A and C).
- Nesprin-1 and Nesprin-2 related muscular dystrophy caused by defects in the *SYNE1* and *SYNE2* gene, respectively; LUMA related muscular dystrophy caused by defects in the *TMEM43* gene; LAP1B related muscular dystrophy caused by defects in the *TOR1AIP1* gene.
- Facio-scapulo-humeral muscular dystrophy, type 1 (FSHD1A), such as associated with defect in the *DUX4* gene (contraction of the D4Z4 macrosatellite repeat in the subtelomeric region of chromosome 4q35) or the *FRG1* gene; Facio-scapulo-humeral muscular dystrophy, type 2 (FSHD1B) caused by defects in the *SMCHD1* gene.
- Spinal muscular atrophy is a genetic disorder caused by mutations in the Survival Motor Neuron 1 (*SMN1*) gene which is characterized by weakness and wasting (atrophy) in muscles used for movement.
- X-linked myotubular myopathy is a genetic disorder caused by mutations in the myotubularin (*MTM1*) gene which affects muscles used for movement (skeletal muscles) and occurs almost exclusively in males. This condition is characterized by muscle weakness (myopathy) and decreased muscle tone (hypotonia).
- Titinopathies are genetic disorders caused by mutations in the Titin (TTN) gene. Both dominant and recessive *TTN* mutations have been reported to cause a wide spectrum of cardiac and skeletal muscle diseases. Dominant titinopathies include hereditary myopathy with early respiratory failure (HMERF) caused by mutations in exon 344, and late-onset tibial muscular dystrophy (TMD). Recessive titinopathies include limb-girdle muscular dystrophy 2J, young- or early-adult-onset distal titinopathy, Emery-Dreifuss-like myopathy without cardiomyopathy, and congenital myopathy with or without heart disease.
- Pompe disease is a genetic disorder caused by mutations in the acid alpha-glucosidase *(GAA)* gene. Mutations in the *GAA* gene prevent acid alpha-glucosidase from breaking down glycogen effectively, which allows this sugar to build up to toxic levels in lysosomes. This buildup damages organs and tissues throughout the body, particularly the muscles, leading to the progressive signs and symptoms of Pompe disease.
- Glycogen storage disease III (GSD3) is an autosomal recessive metabolic disorder caused by homozygous or compound heterozygous mutation in the Amylo-Alpha-1, 6-Glucosidase, 4-Alpha-Glucanotransferase (AGL) gene which encodes the glycogen debrancher enzyme and associated with an accumulation of abnormal glycogen with short outer chains. Clinically, patients with GSD III present in infancy or early childhood with hepatomegaly, hypoglycemia, and growth retardation. Muscle weakness in those with IIIa is minimal in childhood but can become more severe in adults; some patients develop cardiomyopathy.

In some embodiments, the target gene for gene therapy (additive gene therapy or gene editing) is a gene responsible for a neuromuscular disease selected from the group comprising Dystrophinopathies *(DMD* gene) and Limb-girdle muscular dystrophies (LGMDs) *(CAPN3, DYSF, FKRP, ANO5, DNAJB6* genes and others such as *SGCA, SGCB, SGCG).* In some preferred embodiments, the target gene for gene therapy is selected from the group consisting of : *DMD, CAPN3, DYSF, FKRP, DNAJB6, ANO5, SGCA, SGCB* and *SGCG.*

A specific example of gene editing would be the treatment of Limb-girdle muscular dystrophy 2A (LGMD2A) which is caused by mutations in the calpain-3 gene *(CAPN3).* Other non-limiting examples would be the treatment of mutations in the *DMD* or *TNT* genes.

Thus, by gene editing or gene replacement a correct version of this gene is provided in muscle cells of affected patients, this may contribute to effective therapies against this disease. Other genetic diseases of the muscle as listed above could be treated by gene replacement or gene editing using the same principle.

Replacement or additive gene therapy may be used to treat cancer, in particular rhabdomyosarcomas. Genes of interest in cancer could regulate the cell cycle or the metabolism and migration of the tumor cells, or induce tumor cell death. For instance, inducible caspase-9 could be expressed in muscle cells to trigger cell death, preferably in combination therapy to elicit durable anti-tumor immune responses.

Gene editing may be used to modify gene expression in target cells (i.e. muscle cells), in the case of auto-immunity or cancer, or to perturb the cycle of viruses in such cells. In such cases, preferably, the gene of interest is chosen from those encoding guide RNA (gRNA), site-specific endonucleases (TALEN, meganucleases, zinc finger nucleases, Cas nuclease), DNA templates and RNAi components, such as shRNA and microRNA. Tools such as CRISPR/Cas9 may be used for this purpose.

In some embodiments, gene therapy is used for treating diseases affecting other tissues, by expression of a therapeutic gene in muscle tissue. This is useful to avoid expression of the therapeutic gene in the liver, in particular in patients having a concurrent hepatic disorder such as hepatitis, including viral or toxic hepatitis. The therapeutic gene encodes preferably a therapeutic protein, peptide or antibody which is secreted from the muscle cells into the blood stream where it can be delivered to other target tissues such as for example the liver. Examples of therapeutic genes include with no limitation: Factor VIII, Factor IX and GAA genes.

In the various embodiments the pharmaceutical composition comprises a therapeutically effective amount of rAAV vector particle or cell. In the context of the invention a therapeutically effective amount refers to a dose sufficient for reversing, alleviating or inhibiting the progress of the disorder or condition to which such term applies, or reversing, alleviating or inhibiting the progress of one or more symptoms of the disorder or condition to which such term applies. The term "effective dose" or "effective dosage" is defined as an amount sufficient to achieve, or at least partially achieve, the desired effect.

The effective dose is determined and adjusted depending on factors such as the composition used, the route of administration, the physical characteristics of the individual under consideration such as sex, age and weight, concurrent medication, and other factors, that those skilled in the medical arts will recognize.

In the various embodiments of the present invention, the pharmaceutical composition comprises a pharmaceutically acceptable carrier and/or vehicle.

A "pharmaceutically acceptable carrier" refers to a vehicle that does not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

Preferably, the pharmaceutical composition contains vehicles, which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical forms suitable for inj ectable use include sterile aqueous solutions or suspensions. The solution or suspension may comprise additives which are compatible with viral vectors and do not prevent viral vector particle entry into target cells. In all cases, the form must be sterile and must be fluid to the extent that easy syringe ability exists. It must be stable under the conditions of manufacture and storage and must be preserved against the contaminating action of microorganisms, such as bacteria and fungi. An example of an appropriate solution is a buffer, such as phosphate buffered saline (PBS) or Ringer lactate.

The disclosure provides also a method of treating a disease by expression of a therapeutic gene in a target tissue (i.e. muscle tissue), comprising: administering to a patient a therapeutically effective amount of the pharmaceutical composition as described above.

The disclosure provides also a method of treating a muscle disorder, comprising: administering to a patient a therapeutically effective amount of the pharmaceutical composition as described above.

A further aspect relates to the use of a rAAV vector particle, cell, pharmaceutical composition according to the present disclosure in the manufacture of a medicament for the treatment of a muscle disorder, in particular neuromuscular genetic disease according to the present disclosure.

A further aspect relates to a rAAV vector particle, cell, pharmaceutical composition according to the present disclosure as medicament and/or for use in gene therapy, in particular for use in the treatment of a muscle disorder, in particular neuromuscular genetic disease according to the present disclosure.

A further aspect relates to the use of a rAAV vector particle, cell, pharmaceutical composition according to the present disclosure for the treatment of a muscle disorder, in particular neuromuscular genetic disease according to the present disclosure.

A further aspect relates to a pharmaceutical composition for the treatment of a muscle disorder, in particular neuromuscular genetic disease according to the present disclosure, comprising a rAAV vector particle, cell, according to the present disclosure as an active component.

A further aspect relates to a pharmaceutical composition comprising a rAAV vector particle, cell, according to the present disclosure for treating a muscle disorder, in particular neuromuscular genetic disease according to the present disclosure.

As used herein, the term "patient" or "individual" includes human and other mammalian subjects that receive either prophylactic or therapeutic treatment. Preferably, a patient or individual according to the invention is a human.

Treatment", or "treating" as used herein, is defined as the application or administration of a therapeutic agent or combination of therapeutic agents to a patient, or application or administration of said therapeutic agents to an isolated tissue or cell line from a patient, who has a disease, in particular a muscle disorder with the purpose to cure, heal, alleviate, relieve, alter, remedy, ameliorate, improve or affect the disease, or any symptom of the disease. In particular, the terms "treat' or treatment" refers to reducing or alleviating at least one adverse clinical symptom associated with the disease.

The term "treatment" or "treating" is also used herein in the context of administering the therapeutic agents prophylactically.

The pharmaceutical composition of the present invention is generally administered according to known procedures, at dosages and for periods of time effective to induce a therapeutic effect in the patient. The pharmaceutical composition may be administered by any convenient route, such as in a non-limiting manner by infusion or bolus injection, by absorption through epithelial or mucocutaneous linings (e.g., oral mucosa, rectal and intestinal mucosa, etc.). The administration can be systemic, local or systemic combined with local; systemic includes parenteral and oral, and local includes local and loco-regional. Systemic administration is preferably parenteral such as subcutaneous (SC), intramuscular (IM), intravascular such as intravenous (IV) or intraarterial; intraperitoneal (IP); intradermal (ID), epidural or else. The administration may be for example by injection or perfusion. In some preferred embodiments, the administration is parenteral, preferably intravascular such as intravenous (IV) or intraarterial. The parenteral administration is advantageously by injection or perfusion.

The various embodiments of the present disclosure can be combined with each other and the present disclosure encompasses the various combinations of embodiments of the present disclosure.

The practice of the present invention will employ, unless otherwise indicated, conventional techniques, which are within the skill of the art. Such techniques are explained fully in the literature.

The invention will now be exemplified with the following examples, which are not limitative, with reference to the attached drawings in which:

### FIGURE LEGENDS

Figure 1: **Expression level of ITGAV and ITGB6**
   **A-B.** Normalized read (Counts per million - CPM) of ITGAV and ITGB6 in bulk RNA-seq of different human tissues. Data were extracted and reanalysed from GTEx database (https://gtexportal.org/home/)..
Figure 2: **Cap9rh74_VR4_modified's infectivity in human myoblasts and myotubes**
   Luciferase activities 48h-post-infection of 5 AAV variants in human myoblasts and myotubes.
Figure 3: **Cap9rh74_VR4_modified's distribution following intravenous injection in WT** mice.
   Luciferase activities (A) and Vector Copy Number (B) in different tissues (TA, Diaphragm, Quadriceps, Heart, Liver, Lung and Kidney, respectively) in different AAV-injected or control groups.

### EXAMPLE : Construction and biodistribution of the capsid-modified rAAV vector

### 1. Material and Methods

### 1.1. Plasmid construction

Plasmids containing hybrid AAV9.rh74 capsid modified by different peptides containing the motif RGDLXXL/I with XX different from GS and ST (SEQ ID NO: 1) were derived from plasmid pRep2-Cap9rh74 containing AAV2 Rep and hybrid AAV9.rh74 capsid (Cap9rh74 _WT) genes disclosed in WO 2019/193119 using standard cloning procedures. Peptide 4um9 or 4um9 _native (TDGRGDLGRLGP), 4um9_modified (SEPRGDLGELNA) 5ffo or 5ffo_native (EPRRGDLATIGS), 5ffo_modified (TDQRGDLAELHG), 5nem_native (APVRGDLQVLAP) and 5nem_modified (APVRGDLAEINP)were inserted into the variable region 4 (VR-IV) between residues S452 and Q460 of hybrid AAV9.rh74 capsid protein sequence, thereby replacing residues T453 to T459. Bacterial clones were validated by sequencing. The resulting peptide-modified hybrid AAV9.rh74 capsids (VP1 protein) were named Cap9rh74_VR4-4um9 or Cap9rh74_VR4-4um9.Nat (SEQ ID NO: 21), Cap9rh74_VR4-4um9.Mod (SEQ ID NO: 22), Cap9rh74_VR4-5ffo or Cap9rh74_VR4-5ffo.Nat (SEQ ID NO: 23), Cap9rh74_VR4-5ffo.Mod (SEQ ID NO: 24), Cap9rh74_VR4-5nem.Nat (SEQ ID NO: 25) and Cap9rh74_VR4-5nem.Mod (SEQ ID NO: 26). The corresponding coding sequences are SEQ ID NO: 27 to 32. A modified hybrid AAV9.rh74 capsid (Cap9rh74-HB-P1) comprising a peptide P1 (RGDLGLS) flanked by AAA and SG inserted into position 589 (e.g. between N589 and A590) of hybrid AAV9.rh74 capsid sequence disclosed in WO2022/053630 was used for comparison.

### 1.2. rAAV production

Recombinant AAVs (rAAVs) comprising the modified and non-modified AAV9rh74, containing GFP-Luciferase transgene under control of CMV promoter were produced at Généthon as described in Ayuso E. et al. (Hum. Gene Ther. 2014, 25, 977-987). Viral genomes are quantified as described in Rohr et al. (J. Virol. Methods, 2002, 106, 81-88). rAAV titers are expressed as viral genome copy number (vg).

### 1.3 In vivo experiment

Animals were handled according to French and European legislation. The procedures on animals were approved by the local ethics committee and by the Ministry of Higher Education, Research and Innovation (APAFIS#19736). The rAAVs expressing GFP-luciferase were administered to 6 week-old C57BL/6 mice (n=4 per group) at a dose of 1×10¹³ vg/kg, by intravenous (IV) injection. Acquisition of *in vivo* bioluminescence images was done 14 days post-injection. 3 weeks after injection, mice were sacrificed by cervical dislocation and several muscles and organs were sampled : Tibialis anterior, Diaphragm, heart, liver, kidneys and lung.

### 1.4 In vivo bioluminescence

Acquisition of *in vivo* bioluminescence images was done 20 days post-injection. Mice were anesthetized by inhalation of isoflurane and injected intraperitoneally with 100 ul of 50 mg/ml D-luciferin (LifeTechnologies, California, USA). *In vivo* imaging was performed using an IVIS^{®} Lumina Imaging system (PerkinElmer).

### 1.5 Vector copy number quantification

gDNA and viral DNA were extracted from the different muscles and organs sampled using the NucleoMag Pathogen kit (Macherey-Nagel) and the KingFisher Flex instrument (ThermoFisher Scientific).

Viral genome copies were quantified by multiplex qPCR in a Light Cycler 480 Instrument (Roche), using Thermo Scientific Absolute qPCR ROX Mix, primers (Forward: CATCAATGGGCGTGGATAGC (SEQ ID NO: 35); Reverse: GGAGTTGTTACGACATTTTGGAAA (SEQ ID NO: 36)) and probe (ATTTCCAAGTCTCCACCC, FAM (SEQ ID NO: 37)) to detect CMV promoter from the vectors and primers (Forward: CTCCAAGCAGATGCAGCAGA (SEQ ID NO: 38); Reverse: ATAGCCTTGCGCATCATGGT (SEQ ID NO: 39)) and probe (CCGTGGTGCTGATGGGCAAGAA, VIC (SEQ ID NO: 40)) to detect Rplp0 (60S acidic ribosomal protein P0) as internal control of the sample.

### 1.6. In vitro luciferase assay

Samples were homogenized in a lysis buffer (25 mM Tris-phosphate, 15% glycerol, 1mM DTT, 1mM EDTA, 8mM MgCl2, 0.2% Triton X-100 ) with protease inhibitor cocktail (cOmpleteTM ULTRA Tablets, Mini EDTA-free, EASYpack Protease Inhibitor Cocktail Tablets REF:5892791001) using a FastPrep-24 Classic Instrument (MP Biomedicals) (5m/s, 40s). Samples were subjected to 3 freezing/thawing cycles. After centrifugation (5 min, 10000 g, +4°C), 10 ul of supernatant were transferred to a white opaque plate. An EnSpire Multimode Plate Reader (PerkinElmer), with a pumping system that allows dispatching of 100 ul of the assay buffer (same as lysis buffer without Triton X-100 and with 2nM ATP, Sigma, REF: 10519979001) and 100 ul of 167 uM D-Luciferin, was used to measure the luminescence signal from the samples homogenates. Quantification of proteins, to normalize the luminescence signal by the quantity of proteins of the sample, was performed using the Pierce BCA Protein Assay kit.

### 2. Results

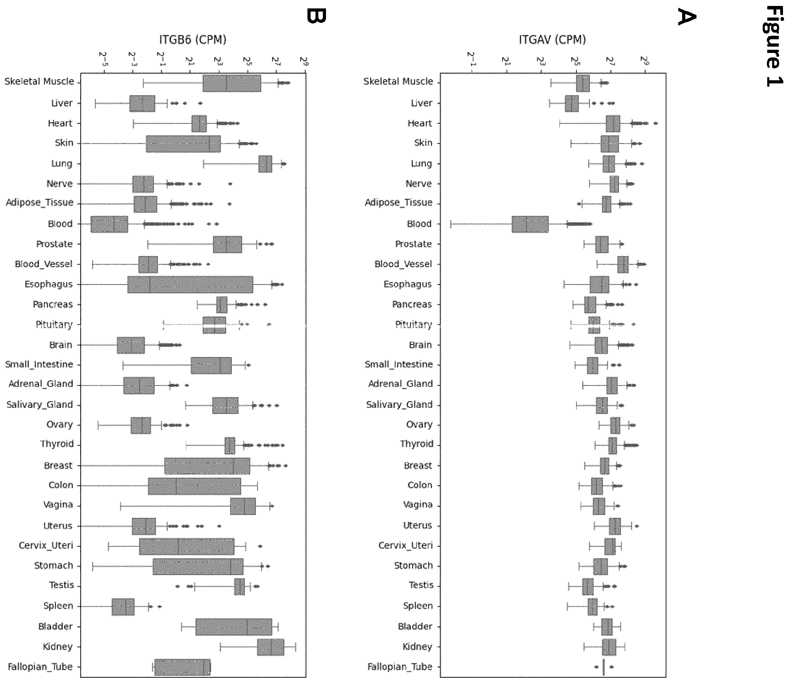

Recombinant AAV vectors targeting the integrin heterodimer αVβ6 (ITGAV-B6) were designed for the following reasons. Firstly, ITGB6 expression is highly upregulated in human skeletal muscle (**Figure 1A-B**). Although not overexpressed in muscle tissue, ITGAV expression in muscle is significantly higher than in the liver, which is of interest for liver-detargeting. Secondly, high expression level of both transcripts in myocyte nuclei and even higher in the dystrophic model - DMD^{delta51} were previously observed by single-nucleus sequencing in mouse muscle tissue (Chemello et al., PNAS, 2020, 117, 29691-29701;). These results are of particular interest for targeting muscle tissues, particularly in muscular dystrophies. Mechanistically, ITGAV-B6 binds with high affinity to RGDLxxL/I motif, in which LxxL/I forms an amphipathic α-helix that binds in a hydrophobic pocket in the B6 subunit. The motifs can be found in the prodomains of TGF-β1, TGF-β3, and FMDV virus capsid, which all specifically bind to ITGAV-B6 (Dong et al., Nature Struct. Mol. Biol., 2014, 21, 1091-1096; Dong et al., Nature, 2017, 542, 55-59; Kotecha et al., Nature communications, 2017, 8, 15408; Protein Data Bank accession number 5FFO,4UM9 and 5NEM).

Capsid-modified rAAV were designed using the hybrid capsid Cap9rh74 as backbone. The whole loop in VR-IV (VR4) region was replaced by 8 amino acids of xRGDLxxL/I motifs obtained from Protein Data Bank: 4UM9, 5FFO, and 5NEM. Suitable linkers (2 amino acids) were added in each end of the peptides to permit a stable conformation of the RGD motif in the VR4 loop. The constructs with the best energy scores were selected for experimental validation using Rosetta energy function. Recombinant AAV vectors containing the modified capsids and a CMV-GFP/Luc transgene expression cassette were produced and purified. All capsid modified rAAVs were produced with good titers (AAV_Cap9rh74_VR4-4um9.Nat: 8.1x10¹³ VG/mL; AAV _Cap9rh74_VR4-4um9.Mod: 1.2x10¹³ VG/mL; AAV_Cap9rh74_VR4-5ffo.Nat: 3x10¹³ VG/mL; AAV_Cap9rh74_VR4-5ffo.Mod: 3.5×10¹² VG/mL; AAV_Cap9rh74_VR4-5nem.Nat: 1.5×10¹² VG/mL; AAV_Cap9rh74_VR4-5nem.Mod: 3.1×10¹³ VG/mL) comparable to that obtained for wild-type AAV_Cap9rh74 (AAV9.rh74: 3.26×10¹³ VG/mL) for most capsid-modified rAAVs.

Next, the binding of the capsid-modified rAAVs to ITGAV-B6 heterodimers was examined. First, a 293 cell line simultaneously overexpressing hITGAV and hITGB6 was constructed using piggyBac^{™} transposon system. Purified capsid-modified rAAVs were then infected in either 293_WT or 293_ITGAV-B6 at 2 different doses, 5E9 and 5E10 vg. All capsid-modified rAAVs showed dose-dependent levels of Luciferase activity, 100-1000 fold higher than wild-type AAV_Cap9rh74.

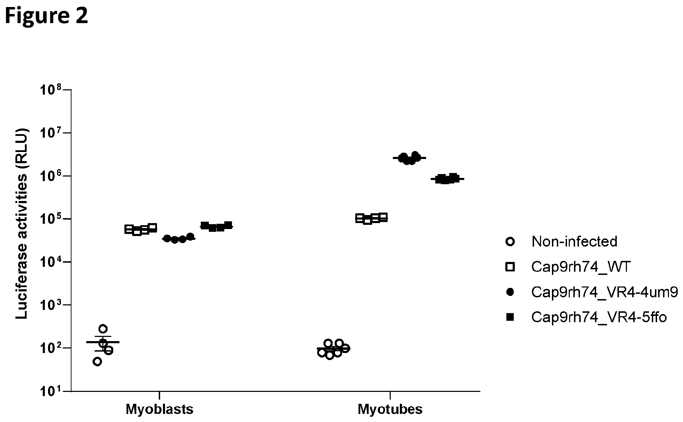

During myogenic differentiation, ITGB6 is highly expressed only at the differentiated myotube stage, but not in satellite cells or myoblasts. To analyse the correlation of ITGAV-B6 expression level and rAAV infectivity in myogenic system, the capsid-modified rAAVs were tested in human myoblasts and myotubes **(****Figure 2****).** As expected, the infection level of all capsid-modified rAAVs is very low at the myoblast stage. All tested capsid-modified rAAVs showed increased infectivity in myotubes, compared to wild-type AAV_Cap9rh74.

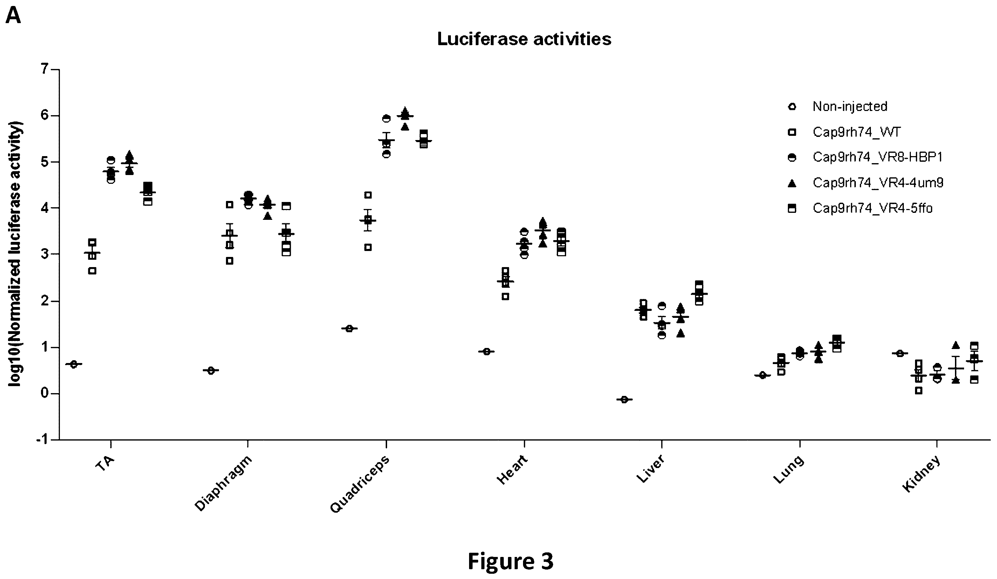

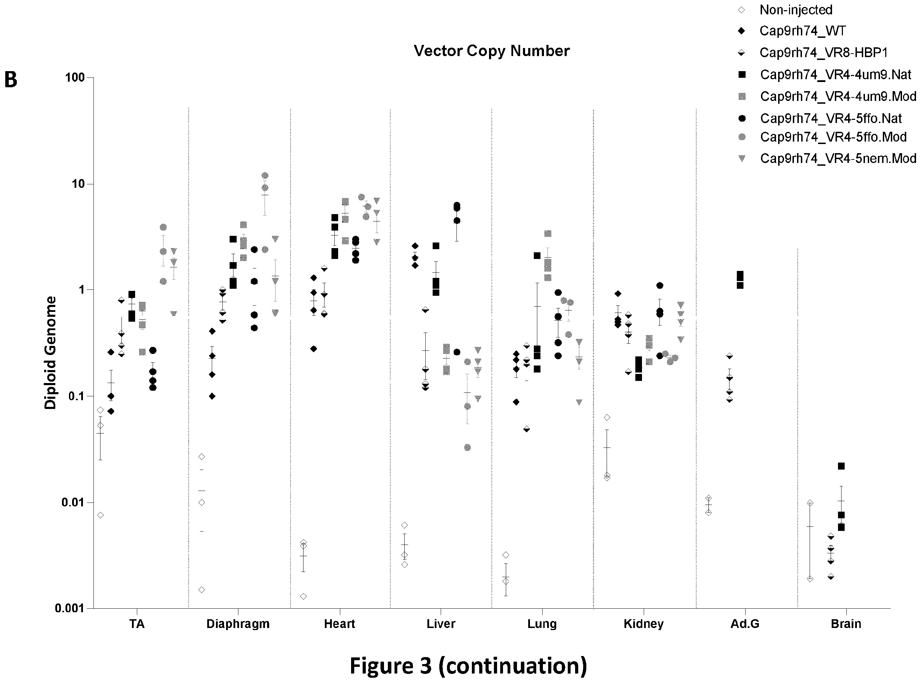

Next, the biodistribution of the capsid-modified rAAVs was tested following intravenous injection in wild-type mice and Vector Copy Number and luciferase activity determination. AAV_Cap9rh74-HB-P1 was also included as positive control for a muscle-enriched and liver-detargeting vector. As shown by the bioluminescent images, all AAV-modified variants are highly enriched in muscle, compared to the Cap9rh74-WT. Furthermore, the luciferase activity and VCN assays confirmed a higher transduction level in all muscles compared to Cap9rh74_WT **(****Figure 3A** **and** **B****).** Cap9rh74_VR4-4um9 increases the transduction level in Quadriceps by 100 fold compared to wild-type Cap9rh74 and around 3 times compared to Cap9rh74-HBP1 **(****Figure 3A** **and Table** 1).

**Table 1: Muscle transduction efficiency of**

| **AAV_Cap9.rh74 modified with RGDXXL/I-peptide** | | | | |
|---|---|---|---|---|
| **AAV vector** | **Fold change of muscle transduction** | | | |
| | **TA** | **Diaphragm** | **Quadriceps** | **Heart** |
| Cap9rh74_VR4-4um9 vs Cap9rh74_WT | 79.34 (p<0.0001) | 2.88 (p=0.0191) | 129.31 (p<0.0001) | 12.37 (p<0.0001) |
| Cap9rh74_VR4-4um9 vs Cap9rh74_VR8-HBP1 | 1.49 (p=0.2441) | 0.74 (p=0.5772) | 2.71 (p=0.0295) | 1.96 (p=0.0804) |
| Cap9rh74_VR4-5ffo vs Cap9rh74_WT | 18.41 (p<0.0001) | 0.98 (p=0.8676) | 37.06 (p<0.0001) | 7.13 (p<0.0001) |
| Cap9rh74_VR4-5ffo vs Cap9rh74_VR8-HBP1 | 0.35 (p=0.0092) | 0.25 (p=0.0090) | 0.78 (p=0.9413) | 1.13 (p=0.6889) |
| * values deduced from the data presented in Figure 3A | | | | |

Using this approach, at least 2 promising candidates (Cap9rh74_VR4-4um9 and Cap9rh74_VR4-5ffo) that were produced with high titers, high affinity with ITGAV-B6 heterodimers, and showed high infectivity in human myotubes as well as high transduction efficiency of muscles were produced.

### List of sequences

| **SEQ ID NO:** | **Sequence name** |
|---|---|
| **1** | ITGAV-B6 binding motif |
| **2** | Peptide 4um9(4um9 native) |
| **3** | Peptide 4um9 modified |
| **4** | Peptide 5off |
| **5** | Peptide 5off modified |
| **6** | Peptide 5nem |
| **7** | Peptide 5nem modified |
| **8** | Peptide 4um9 +G |
| **9** | Peptide 4um9 modified + P |
| **10** | Peptide 5off + R |
| **11** | Peptide 5off modified + Q |
| **12** | Peptide 5nem + V |
| **13** | Peptide 5nem modified + V |
| **14** | Peptide 4um9 + linker |
| **15** | Peptide 4um9 modified + linker |
| **16** | Peptide 5off + linker |
| **17** | Peptide 5off modified + linker |
| **18** | Peptide 5nem linker |
| **19** | Peptide 5nem modified + linker |
| **20** | AAV9.rh74 capsid (VP1 protein) |
| **21** | Cap9rh74_VR4-4um9(LICA.01) (VP1 protein) |
| **22** | Cap9rh74_VR4-4um9_modified(VP1 protein) |
| **23** | Cap9rh74_VR4-5ffo(VP1 protein) |
| **24** | Cap9rh74_VR4-5ffo modified(VP1 protein) |
| **25** | Cap9rh74_VR4-5nem(VP1 protein) |
| **26** | Cap9th74_VR4-5nem_modified(VP1 protein) |
| **27** | Cap9rh74_VR4-4um9(VP1 CDS) |
| **28** | Cap9rh74_VR4-4um9 modified(VP1 CDS) |
| **29** | Cap9rh74 VR4-5ffo(VP1 CDS) |
| **30** | Cap9rh74_VR4-5ffo modified(VP1 CDS) |
| **31** | Cap9rh74_VR4-nem (VP1 CDS) |
| **32** | Cap9rh74_VR4-nem modified(VP1 CDS) |
| **33** | Peptide P1 |
| **34** | Cap9rh74-HB-P1 (VP1 protein) |
| **35** | CMV-FW primer |
| **36** | CMV-RV primer |
| **37** | CMV-probe (FAM) |
| **38** | Rplp0-FW primer |
| **39** | Rplp0-RV primer |
| **40** | Rplp0-probe (VIC) |
| **41** | Peptide MyoAAV 1A |
| **42** | Peptide MyoAAV 2A |
| **43** | Peptide MyoAAV 4A |
| **44** | Peptide MyoAAV 4E |
| **45** | Peptide MyoAAV 3A |
| **46** | Peptide MyoAAV 4C |

## Claims

1. A recombinant adeno-associated virus (AAV) capsid protein, comprising the insertion of at least one copy of a peptide comprising the motif RGDLXXLII with XX different from GS and ST (SEQ ID NO: 1) which binds to the integrin heterodimer alpha-V beta-6 (ITGAV-B6), wherein said recombinant AAV capsid protein modified with the peptide has an increased transduction efficiency in muscle compared to the recombinant AAV capsid protein not modified with the peptide.

2. The recombinant AAV capsid protein of claim 1, wherein the insertion is into the variable region IV.

3. The recombinant AAV capsid protein of claim 1 or claim 2, which is a hybrid between AAV serotype 9 (AAV9) and AAV serotype 74 (AAVrh74) capsid proteins.

4. The recombinant AAV capsid protein of claim 2 or 3, wherein the insertion is into position 452; preferably wherein the peptide insertion replaces the residues from positions 453 to 459, and the indicated positions being determined by alignment with SEQ ID NO: 20.

5. The recombinant AAV capsid protein of any one of claims 1 to 4, wherein said motif is RGDLX₁X₂L/I wherein X₁ is G, A, L, K or Q and X₂ is R, K, E, D, A, L, T, I or V.

6. The recombinant AAV capsid protein according to claim 5, wherein said motif has a sequence selected from the group consisting of: RGDLGRL (SEQ ID NO: 2), RGDLGEL (SEQ ID NO: 3), RGDLATI (SEQ ID NO: 4), RGDLAEL (SEQ ID NO: 5), RGDLQVL (SEQ ID NO: 6) and RGDLAEI (SEQ ID NO: 7); preferably SEQ ID NO: 2 or SEQ ID NO: 4; more preferably SEQ ID NO: 2.

7. The recombinant AAV capsid protein of claim 6, wherein said peptide comprises or consists of a sequence selected from the group consisting of: TDGRGDLGRLGP (SEQ ID NO: 14), SEPRGDLGELNA (SEQ ID NO: 15), EPRRGDLATIGS (SEQ ID NO: 16), TDQRGDLAELHG (SEQ ID NO: 17), APVRGDLQVLAP (SEQ ID NO: 18) and APVRGDLAEINP (SEQ ID NO: 19); preferably SEQ ID NO: 14 or 16; more preferably SEQ ID NO: 14.

8. The recombinant AAV capsid protein of claim 7, which comprises a sequence having at least 85% identity with any one of SEQ ID NO: 21 to 26; preferably SEQ ID NO: 21 or 23; more preferably SEQ ID NO: 21.

9. A polynucleotide encoding the recombinant AAV capsid protein according to any one of claims 1 to 8.

10. A recombinant plasmid comprising the polynucleotide of claim 9.

11. An AAV vector particle packaging a gene of interest, which comprises the recombinant AAV capsid protein according to any one of claims 1 to 8.

12. The AAV vector particle according to claim 11, wherein the gene of interest is selected from the group consisting of: therapeutic genes; genes encoding therapeutic proteins or peptides such as therapeutic antibodies or antibody fragments and genome editing enzymes; and genes encoding therapeutic RNAs such as interfering RNAs, guide RNAs for genome editing and antisense RNAs capable of exon skipping.

13. A pharmaceutical composition comprising a therapeutically effective amount of an AAV vector particle according to claim 11 or 12, or a cell stably transduced by said AAV vector particle of claim 11 or 12.

14. The pharmaceutical composition of claim 13, for use as a medicament in gene therapy.

15. The pharmaceutical composition of claim 13, for use in the treatment of muscle diseases.

16. The pharmaceutical composition for use of claim 15, which targets a gene responsible for a muscle disease chosen from Dystrophinopathies and Limb-girdle muscular dystrophies; preferably a gene selected from the group comprising: *DMD, CAPN3, DYSF, FKRP, DNAJB6, ANO5, SGCA, SGCB* and *SGCG.*

## Patentansprüche

1. Rekombinantes Adeno-assoziiertes Virus (AAV)-Kapsidprotein, umfassend die Insertion mindestens einer Kopie eines Peptids, das das Motiv RGDLXXL/I umfasst, wobei XX von GS und ST verschieden ist (SEQ ID NO: 1), das an das Integrin-Heterodimer Alpha-V Beta-6 (ITGAV-B6) bindet, wobei das mit dem Peptid modifizierte, rekombinante AAV-Kapsidprotein im Vergleich zu dem nicht mit dem Peptid modifizierten rekombinanten AAV-Kapsidprotein eine erhöhte Transduktionseffizienz im Muskel aufweist.

2. Rekombinantes AAV-Kapsidprotein nach Anspruch 1, wobei die Insertion in die variable Region IV erfolgt.

3. Rekombinantes AAV-Kapsidprotein nach Anspruch 1 oder Anspruch 2, das ein Hybrid zwischen AAV-Serotyp 9 (AAV9) und AAV-Serotyp 74 (AAVrh74) Kapsidproteinen ist.

4. Rekombinantes AAV-Kapsidprotein nach Anspruch 2 oder 3, wobei die Insertion an Position 452 erfolgt; vorzugsweise wobei die Peptidinsertion die Reste von Position 453 bis 459 ersetzt und die angegebenen Positionen durch Ausrichtung mit SEQ ID NO: 20 bestimmt werden.

5. Rekombinantes AAV-Kapsidprotein nach einem der Ansprüche 1 bis 4, wobei das Motiv RGDLX₁X₂L/I ist, wobei X₁ G, A, L, K oder Q ist und X₂ R, K, E, D, A, L, T, I oder V ist.

6. Rekombinantes AAV-Kapsidprotein nach Anspruch 5, wobei das Motiv eine Sequenz aufweist, die aus der Gruppe ausgewählt ist, die aus RGDLGRL (SEQ ID NO: 2), RGDLGEL (SEQ ID NO: 3), RGDLATI (SEQ ID NO: 4), RGDLAEL (SEQ ID NO: 5), RGDLQVL (SEQ ID NO: 6) und RGDLAEI (SEQ ID NO: 7) besteht; vorzugsweise SEQ ID NO: 2 oder SEQ ID NO: 4; stärker bevorzugt SEQ ID NO: 2.

7. Rekombinantes AAV-Kapsidprotein nach Anspruch 6, wobei das Peptid eine Sequenz umfasst oder aus einer Sequenz besteht, die aus der Gruppe ausgewählt ist, die aus TDGRGDLGRLGP (SEQ ID NO: 14), SEPRGDLGELNA (SEQ ID NO: 15), EPRRGDLATIGS (SEQ ID NO: 16), TDQRGDLAELHG (SEQ ID NO: 17), APVRGDLQVLAP (SEQ ID NO: 18) und APVRGDLAEINP (SEQ ID NO: 19) besteht; vorzugsweise SEQ ID NO: 14 oder 16; stärker bevorzugt SEQ ID NO: 14.

8. Rekombinantes AAV-Kapsidprotein nach Anspruch 7, das eine Sequenz umfasst, die mindestens 85 % Identität mit einer der Sequenzen SEQ ID NO: 21 bis 26 aufweist, vorzugsweise SEQ ID NO: 21 oder 23, stärker bevorzugt SEQ ID NO: 21.

9. Polynukleotid, das das rekombinante AAV-Kapsidprotein nach einem der Ansprüche 1 bis 8 codiert.

10. Rekombinantes Plasmid, das das Polynukleotid nach Anspruch 9 umfasst.

11. AAV-Vektorpartikel, das ein Gen von Interesse verpackt, das das rekombinante AAV-Kapsidprotein nach einem der Ansprüche 1 bis 8 umfasst.

12. AAV-Vektorpartikel nach Anspruch 11, wobei das Gen von Interesse aus der Gruppe ausgewählt ist, die besteht aus: therapeutischen Genen; Genen, die therapeutische Proteine oder Peptide wie therapeutische Antikörper oder Antikörperfragmente und Genom-Editierungsenzyme kodieren; und Genen, die therapeutische RNAs wie interferierende RNAs, Leit-RNAs für die Genom-Editierung und Antisense-RNAs, die zum Exon-Skipping fähig sind, kodieren.

13. Pharmazeutische Zusammensetzung, umfassend eine therapeutisch wirksame Menge eines AAV-Vektorpartikels nach Anspruch 11 oder 12 oder eine Zelle, die durch das AAV-Vektorpartikel nach Anspruch 11 oder 12 stabil transduziert ist.

14. Pharmazeutische Zusammensetzung nach Anspruch 13 zur Verwendung als Medikament in der Gentherapie.

15. Pharmazeutische Zusammensetzung nach Anspruch 13 zur Verwendung bei der Behandlung von Muskelerkrankungen.

16. Pharmazeutische Zusammensetzung zur Verwendung nach Anspruch 15, die auf ein Gen abzielt, das für eine Muskelerkrankung verantwortlich ist, ausgewählt aus Dystrophinopathien und Gliedergürtel-Muskeldystrophien; vorzugsweise ein Gen, ausgewählt aus der Gruppe bestehend aus: *DMD, CAPN3, DYSF, FKRP, DNAJB6, AN05, SGCA, SGCB* und SGCG.

## Revendications

1. Protéine capsidique de virus adéno-associé (AAV) recombinante, comprenant l'insertion d'au moins une copie d'un peptide comprenant le motif RGDLXXL/I avec XX différent de GS et ST (SEQ ID NO: 1) qui se lie à l'hétérodimère alpha-V bêta-6 d'intégrine (ITGAV-B6), dans laquelle ladite protéine capsidique d'AAV recombinante modifiée avec le peptide présente une efficacité de transduction accrue dans le muscle par rapport à la protéine capsidique d'AAV recombinante non modifiée avec le peptide.

2. Protéine capsidique d'AAV recombinante selon la revendication 1, dans laquelle l'insertion se situe dans la région variable IV.

3. Protéine capsidique d'AAV recombinante selon la revendication 1 ou la revendication 2, qui est un hybride entre des protéines capsidiques d'AAV de sérotype 9 (AAV9) et d'AAV de sérotype 74 (AAVrh74).

4. Protéine capsidique d'AAV recombinante selon la revendication 2 ou 3, dans laquelle l'insertion se situe en position 452 ; de préférence, dans laquelle l'insertion du peptide remplace les résidus des positions 453 à 459, et les positions indiquées étant déterminées par alignement avec la SEQ ID NO : 20.

5. Protéine capsidique d'AAV recombinante selon l'une quelconque des revendications 1 à 4, dans laquelle ledit motif est RGDLX₁X₂L/I, où X₁ est G, A, L, K ou Q et X₂ est R, K, E, D, A, L, T, I ou V.

6. Protéine capsidique d'AAV recombinante selon la revendication 5, dans laquelle ledit motif présente une séquence choisie dans le groupe consistant en : RGDLGRL (SEQ ID NO : 2), RGDLGEL (SEQ ID NO : 3), RGDLATI (SEQ ID NO : 4), RGDLAEL (SEQ ID NO: 5), RGDLQVL (SEQ ID NO: 6) et RGDLAEI (SEQ ID NO : 7) ; de préférence SEQ ID NO : 2 ou SEQ ID NO : 4 ; de préférence encore SEQ ID NO : 2.

7. Protéine capsidique d'AAV recombinante selon la revendication 6, dans laquelle ledit peptide comprend ou consiste en une séquence sélectionnée dans le groupe consistant en: TDGRGDLGRLGP (SEQ ID NO : 14), SEPRGDLGELNA (SEQ ID NO: 15), EPRRGDLATIGS (SEQ ID NO: 16), TDQRGDLAELHG (SEQ ID NO : 17), APVRGDLQVLAP (SEQ ID NO : 18) et APVRGDLAEINP (SEQ ID NO : 19) ; de préférence SEQ ID NO : 14 ou 16 ; de préférence encore SEQ ID NO : 14.

8. Protéine capsidique d'AAV recombinante selon la revendication 7, qui comprend une séquence ayant au moins 85 % d'identité avec l'une quelconque des SEQ ID NO : 21 à 26; de préférence SEQ ID NO : 21 ou 23 ; de préférence encore SEQ ID NO : 21.

9. Polynucléotide codant pour la protéine capsidique d'AAV recombinante selon l'une quelconque des revendications 1 à 8.

10. Plasmide recombinant comprenant le polynucléotide selon la revendication 9.

11. Particule vecteur AAV renfermant un gène d'intérêt, comprenant la protéine capsidique d'AAV recombinante selon l'une quelconque des revendications 1 à 8.

12. Particule vecteur AAV selon la revendication 11, dans laquelle le gène d'intérêt est sélectionné dans le groupe consistant en : des gènes thérapeutiques ; des gènes codant des protéines ou peptides thérapeutiques tels que des anticorps thérapeutiques ou fragments d'anticorps, ainsi que des enzymes d'édition du génome ; et des gènes codant des ARN thérapeutiques tels que des ARN interférents, des ARN guides pour l'édition du génome et des ARN antisens capables de sauter des exons.

13. Composition pharmaceutique comprenant une quantité thérapeutiquement efficace d'une particule vecteur AAV selon la revendication 11 ou 12, ou une cellule transduite de manière stable par ladite particule vecteur AAV selon la revendication 11 ou 12.

14. Composition pharmaceutique selon la revendication 13, pour son utilisation en tant que médicament en thérapie génique.

15. Composition pharmaceutique selon la revendication 13, pour son utilisation dans le traitement des maladies musculaires.

16. Composition pharmaceutique pour son utilisation selon la revendication 15, qui cible un gène responsable d'une maladie musculaire choisie parmi les dystrophinopathies et les dystrophies musculaires des ceintures ; de préférence un gène sélectionné dans le groupe comprenant : *DMD, CAPN3, DYSF, FKRP, DNAJB6, ANO5, SGCA, SGCB* et *SGCG.*
